(19) Europäisches Patentamt
European Patent Office
Office européen des brevets

(11) Numéro de publication: **0 008 596**
**A1**

(12) # DEMANDE DE BREVET EUROPEEN

(21) Numéro de dépôt: 78400088.7

(51) Int. Cl.³: C 07 D 233/64

(22) Date de dépôt: 04.09.78

(43) Date de publication de la demande: 19.03.80 Bulletin 80/6

(84) Etats Contractants Désignés: BE CH DE FR GB LU NL

(71) Demandeur: Société anonyme dite: DEVINTER Europe S.A.
Place du Champ de Mars 2
B-1050 Bruxelles(BE)

(72) Inventeur: Gignier, Jean Pierre
4 rue des Capucines
F-92190 Meudon(FR)

(72) Inventeur: Bourrely, Jacques
4 avenue du Chateau
F-92190 Meudon(FR)

(74) Mandataire: de Haas, Michel et al,
Cabinet Beau de Lomenie 55 rue d'Amsterdam
F-75000 Paris 8ème(FR)

(54) **Procédé de préparation de dérivés du type 4-hydroxyméthyl imidazole à partir d'esters d'acides 4-imidazole carboxyliques correspondants.**

(57) La présente invention concerne un procédé de préparation de dérivés de formule:

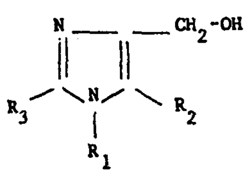

dans laquelle $R_1$, $R_2$ et $R_3$ identiques ou différents sont choisis parmi l'hydrogène et les radicaux alkyles comportant 1 à 4 atomes de carbone par réduction des esters carboxyliques correspondants caractérisé en ce que l'on utilise comme agent réducteur un produit choisi parmi les trialkyle aluminium, les hydrures d'alkyle aluminium et les hydrures d'alcoxy aluminium, comportant ou non un atome de métal alcalin, ladite réaction de réduction étant réalisée en un milieu inerte sec, puis que l'on hydrolyse le produit obtenu par de l'eau ou un mélange eau-alcool.

EP 0 008 596 A1

Croydon Printing Company Ltd.

Procédé de préparation de dérivés du type
4-hydroxyméthyl imidazole à partir d'esters d'acides
4-imidazole carboxyliques correspondants.

La présente invention concerne un procédé de préparation de dérivés du type 4-hydroxyméthyl imidazole à partir d'esters d'acides 4-imidazole carboxyliques correspondants.

Les dérivés du type 4-hydroxyméthyl imidazole de formule :

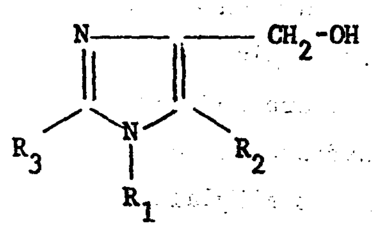

(I)

dans laquelle $R_1$, $R_2$ et $R_3$ identiques ou différents sont choisis parmi l'hydrogène et les radicaux alkyle comportant de 1 à 4 atomes de carbone sont des matières premières intéressantes notamment dans le domaine de la synthèse des produits pharmaceutiques.

La présente invention concerne un procédé de préparation des produits de formule (I) par réduction des esters d'acides 4-imidazole carboxyliques correspondants de formule :

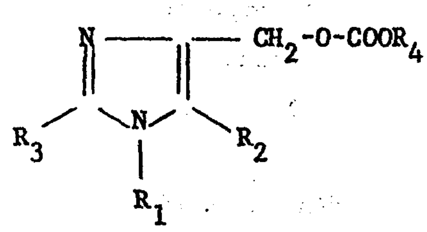

(II)

ledit procédé étant caractérisé en ce que l'on utilise comme réducteur un produit choisi parmi les trialkyl aluminium et les hydrures d'alkyl aluminium.

Les alkyl aluminium sont des produits connus utilisables notamment dans les systèmes catalytiques du type Ziegler ; les propriétés

réductrices de ces produits ont également été décrites ; il a été trouvé que certains de ces alkyl aluminium présentaient des propriétés réductrices particulièrement adaptées à la réduction des esters de formule (II) en dérivés à fonction alcool de formule (I). Les alkyl aluminium utilisables dans la présente invention sont :

- les trialkyl aluminium c'est-à-dire les produits dans lesquels l'aluminium est lié à des radicaux alkyles identiques ou différents,

- les alkyl hydrure d'aluminium c'est-à-dire les produits dans lesquels l'aluminium est lié à au moins un radical alkyle et à au moins un atome d'hydrogène,

- et les dérivés ci-dessus comportant un atome alcalin, de préférence le sodium.

D'autre part on peut utiliser les hydrures de dialcoxy aluminium comportant ou non un atome alcalin (de préférence le sodium).

On citera plus particulièrement :

- l'hydrure sodique de bis-méthoxy aluminium
- l'hydrure sodique de bis-(2-méthoxy éthoxy) aluminium.

Parmi les alkylaluminium et hydrure d'alkyl aluminium utilisables, on citera plus particulièrement :

- le triéthyl aluminium,
- le triisobutyl aluminium,
- le diisobutyl aluminium hydrure,
- l'hydrure de sodium diéthyl aluminium.

La réaction de réduction selon l'invention est effectuée de préférence dans un milieu inerte sec constitué par exemple par un hydrocarbure ou un mélange d'hydrocarbure, notamment de nature aromatique ; la réaction est effectuée à température voisine de l'ambiante, par exemple entre 10 et 70°C et les quantités de réactifs sont choisies de préférence de façon qu'il y ait un léger excès de réducteur par rapport à l'ester traité.

A la fin de la réaction on obtient un complexe aluminique du dérivé d'imidazole que l'on soumet à une hydrolyse pour pouvoir recueillir le dérivé d'imidazole. Cette hydrolyse s'effectue de façon connue à l'aide d'une certaine quantité d'eau mais il est souhaitable d'utiliser, avec ou après l'eau de l'hydrolyse, une certaine quantité d'alcool notamment un alcool aliphatique comportant 1 à 4 atomes de carbone qui permet la séparation de l'imidazole d'avec l'alumine précipitée dans l'hydrolyse.

Le dérivé 4-hydroxyméthyl imidazole est obtenu à l'état très pur, c'est-à-dire sans produits de réaction secondaires, avec un bon rendement ; il peut être isolé ou utilisé directement comme réactif ; il peut notamment être utilisé directement pour réaliser une réaction avec un produit de formule :

$$HS-(CH_2)_n-\underset{R_5}{\underset{|}{CH}}-NH_2 \text{ , } HX$$

dans laquelle n est un nombre entier au moins égal à 1 et $R_5$ est un atome d'hydrogène ou un radical monofonctionnel notamment un radical alkyle ou ester carboxylique, X étant un halogène,
pour former, par condensation, des dérivés intéressants de formule

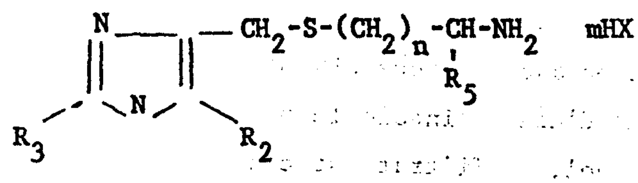

$R_1$, $R_2$, $R_3$, $R_5$, n et X ayant les significations données précédemment et m étant égal à 1 ou 2.

Les exemples non limitatifs suivants illustrent l'invention :

Exemple 1

7,7 g de 5-méthyl-4-carbéthoxy imidazole sont mis en suspension dans 100 ml de toluène anhydre et chauffés à 50°C sous atmosphère d'azote. On introduit 170 ml d'une solution toluénique à 20 % de diisobutyl aluminium hydrure en une heure. Le mélange est agité trois heures à 50°C. La suspension est refroidie à 10°C. L'excès d'agent réducteur est détruit par addition de 20 ml d'éthanol puis de 20 ml d'eau.

La suspension est chauffée à 70°C puis filtrée. Les sels d'aluminium sont lavés à l'éthanol.

Après évaporation des solvants, on récupère 5 g de cristaux blancs de 4-hydroxy méthyl-5 méthyl imidazole. Rendement : 89,3 %. F. 138°C.

Exemple 2

16,8 g de 1-5 diméthyl-4-carbéthoxy imidazole et 250 ml de xylène sont séchés par distillation azéotropique. Le mélange est refroidi à 60°C et 340 ml de solution de triisobutyl aluminium à 25 % dans l'hexane sont introduits en 1 heure 30 environ. Le milieu est maintenu encore 4 à 5 heures à 60°C après la fin d'addition du réactif.

On refroidit le milieu vers 10-15°C puis on ajoute avec précaution 50 ml d'éthanol puis 45 ml d'eau. Les sels insolubles sont

filtrés puis lavés par 20 ml d'éthanol. Le filtrat est concentré puis repris par 175 ml d'acide acétique glacial. On ajoute 17,2 g de chlorhydrate de cystéinate de méthyle. Le mélange obtenu est chauffé pendant 12 heures à 110°C, sous léger barbotage d'acide chlorhydrique gazeux. Par refroidissement le milieu cristallise, et permet après essorage de récupérer 25,8 g de cristaux jaune pâle de 1-5 diméthyl-4 [(2 amino-2-carboxyméthyl-éthyl)thiométhyl]imidazole chlorhydrate. Rendement : 92 %.

Exemple 3

On met en suspension 10 g de 5 méthyl-4 carbéthoxy imidazole dans 135 ml de toluène. Après séchage azéotropique du milieu, le mélange est amené vers 55-60°C puis 265 ml de solution de diisobutyl aluminium hydrure à 20 % dans l'hexane sont introduits en 2 heures.

Après 4 heures de contact à 60°C, le milieu est refroidi à 15°C et on ajoute successivement avec précaution 35 ml d'isopropanol puis 25 ml d'eau. L'insoluble minéral est filtré puis lavé par 20 ml d'isopropanol. Le filtrat est concentré et le résidu repris par 200 ml d'acide propionique. Après addition de 7,4 g de chlorhydrate de cystéamine le mélange est porté à 120°C pendant 15 heures.

Le milieu est concentré au demi de son volume initial puis dilué par 75 ml de méthylisobutylcétone. Après barbotage d'acide chlorhydrique gazeux, on isole par filtration 14,35 g de cristaux blancs de 5 méthyl-4[(2 aminoéthyl)thiométhyl]imidazole bichlorhydrate de pureté supérieure à 99 %. Rendement : 90,5 %.

Exemple 4

On met en suspension 45,3 g de 5 méthyl-4-carbéthoxy imidazole dans 135 ml de toluène.

Après séchage azéotropique le mélange est refroidi à 20°C. Sous atmosphère d'azote on introduit lentement 170 g de solution à 70 % d'hydrure sodique de bis(2 méthoxyéthoxy)aluminium.

A la fin de l'introduction (durée 30 minutes), la température atteint 60°C. On chauffe encore une heure à 80°C puis refroidit à 10°C. On ajoute alors lentement 40 ml d'eau et filtre le précipité.

Les sels minéraux sont éliminés par deux lavages successifs de 10 ml de méthanol.

Après décantation et élimination de la phase organique, on concentre la phase aqueuse jusqu'à un volume résiduel de 35 ml.

On ajoute 150 ml d'acide acétique et on additionne 28,4 g de chlorhydrate de cystéamine, puis du HCl gazeux jusqu'à neutralisation complète.

L'eau formée est éliminée par distillation azéotropique. On refroidit à 0°C. Les cristaux sont lavés et séchés. Poids obtenu : 50 g (70 % de la théorie).

REVENDICATIONS

1. Procédé de préparation de dérivés de formule :

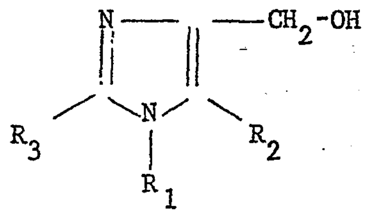

dans laquelle $R_1$, $R_2$ et $R_3$ identiques ou différents sont choisis parmi l'hydrogène et les radicaux alkyles comportant 1 à 4 atomes de carbone par réduction des esters carboxyliques correspondants caractérisés en ce que l'on utilise comme agent réducteur un produit choisi parmi les trialkyle aluminium, les hydrures d'alkyle aluminium et les hydrures d'alcoxy aluminium, comportant ou non un atome de métal alcalin.

2. Procédé selon la revendication 1, caractérisé en ce que l'agent réducteur est le triéthyle ou le triisobutyle aluminium.

3. Procédé selon la revendication 1, caractérisé en ce que l'agent réducteur est un monohydrure de dialkyle aluminium.

4. Procédé selon la revendication 1, caractérisé en ce que l'agent réducteur est un hydrure de sodium dialkyle aluminium.

5. Procédé selon la revendication 1, caractérisé en ce que l'agent réducteur est un hydrure de sodium dialcoxy aluminium.

6. Procédé selon l'une des revendications 1, 2 et 3, caractérisé en ce que la réaction de réduction est réalisée dans un milieu inerte sec tel qu'un hydrocarbure ou un mélange d'hydrocarbure.

7. Procédé selon la revendication 6, caractérisé en ce que le milieu de réaction est du toluène, au moins un xylène ou un mélange de solvants aromatiques.

8. Procédé selon la revendication 6, caractérisé en ce que le milieu de réaction est un hydrocarbure aliphatique ou une coupe pétrolière aliphatique.

9. Procédé selon l'une des revendications précédentes, caractérisé en ce que, après la réaction de réduction, le complexe obtenu est hydrolysé.

10. Procédé selon la revendication 9, caractérisé en ce que l'hydrolyse est effectuée par addition d'eau.

11. Procédé selon la revendication 9, caractérisé en ce que l'hydrolyse est réalisée par un mélange d'eau et d'un alcool aliphatique

comportant de 1 à 4 atomes de carbone.

12. Procédé selon la revendication 9, caractérisé en ce qu'après hydrolyse les dérivés minéraux précipités sont séparés et que l'on recueille le 4-hydroxyméthyle imidazole sous la forme de la base ou du chlorhydrate.

13. Procédé selon la revendication 12, caractérisé en ce que le chlorhydrate de l'imidazole est obtenu par acidification à l'aide d'acide chlorhydrique gazeux.

14. Procédé selon l'une des revendications précédentes, caractérisé en ce que le 4-hydroxyméthyle imidazole obtenu est condensé directement avec un composé de formule générale :

$$HS-(CH_2)_n-\underset{\displaystyle R_5}{\underset{|}{CH}}-NH_2\ ,\ HX$$

dans laquelle n est un nombre entier au moins égal à 1, $R_5$ est un atome d'hydrogène ou un radical monofonctionnel simple notamment un ester carboxylique, X est un halogène.

0008596

Office européen des brevets

# RAPPORT DE RECHERCHE EUROPEENNE

Numéro de la demande
EP 78 40 0088

| DOCUMENTS CONSIDERES COMME PERTINENTS | | | CLASSEMENT DE LA DEMANDE (Int. Cl.²) |
|---|---|---|---|
| Catégorie | Citation du document avec indication, en cas de besoin, des parties pertinentes | Revendication concernée | |
| A | FR - A - 2 319 341 (SKF)<br>* Pages 3,5, lignes 10-21 *<br>-- | 1 | C 07 D 233/64 |
| A | GB - A - 1 341 376 (SKF)<br>* Exemple *<br>---- | 1 | |

**DOMAINES TECHNIQUES RECHERCHES (Int. Cl.²)**

C 07 D 233/64

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 26-06-1979 | DE BUYSER |